# EUROPÄISCHE PATENTSCHRIFT

(11) **EP 1 114 026 B1**
(45) Veröffentlichungstag und Bekanntmachung des Hinweises auf die Patenterteilung: **18.12.2002**
(21) Anmeldenummer: 99944635.4
(22) Anmeldetag: 10.09.1999
(51) Int. Cl.: C07D 201/08, C07D 201/02, C07D 295/02

(54) **VERFAHREN ZUR GLEICHZEITIGEN HERSTELLUNG EINES CYCLISCHEN LACTAMS UND EINES CYCLISCHEN AMINS**
METHOD FOR SIMULTANEOUSLY PRODUCING A CYCLIC LACTAM AND A CYCLIC AMINE
PROCEDE DE PREPARATION SIMULTANEE D'UN LACTAME CYCLIQUE ET D'UNE AMINE CYCLIQUE

(30) Priorität: 18.09.1998 DE 19842905
(43) Veröffentlichungstag der Anmeldung: 11.07.2001
(73) Patentinhaber: BASF AKTIENGESELLSCHAFT, 67056 Ludwigshafen (DE)
(72) Erfinder: MERGER, Martin, D-67227 Frankenthal (DE); FISCHER, Rolf, D-69121 Heidelberg (DE); ANSMANN, Andreas, D-69168 Wiesloch (DE)
(86) Internationale Anmeldenummer: EP9906691
(87) Internationale Veröffentlichungsnummer: WO00017160

(56) Entgegenhaltungen:
- WO-A-96/22974
- DE-A- 19 500 222
- DE-C- 765 203
- DE-C- 915 568

## Beschreibung

Die vorliegende Erfindung betrifft ein Verfahren zur gleichzeitigen Herstellung eines cyclischen Lactams und eines cyclischen Amins durch gleichzeitige Umsetzung eines aliphatischen alpha, omega-Diamins und eines aliphatischen alpha, omega Aminonitrils in der Gasphase mit Wasser in der Gegenwart eines heterogenen Katalysators, wobei das Molverhältnis von Wasser zu der Summe aus aliphatischem alpha, omega-Diamin und aliphatischem alpha, omega-Aminonitril 3 bis 25 ist, das molare Verhältnis von dem aliphatischen alpha, omega-Aminonitril zu dem aliphatischen alpha, omega-Diamin zwischen 5 : 95 und 90 : 10 liegt und der heterogene Katalysator
(a) eine Dehydrierkomponente und
(b) eine acide und/oder amphotere Komponente
enthält.

Die Herstellung von Mischungen enthaltend ein aliphatisches alpha, omega-Diamin und ein aliphatisches alpha, omega-Aminonitril durch partielle Hydrierung eines aliphatischen alpha, omega-Dinitrils, beispielsweise die Herstellung von Mischungen enthaltend Hexamethylendiamin und 6-Aminocapronitril durch partielle Hydrierung von Adipodinitril, ist allgemein bekannt. Die Aufarbeitung solcher Mischungen zur Gewinnung des Diamins und des Aminonitrils ist nur unter erheblichem technischem Aufwand möglich.

Cyclische Lactame, wie Caprolactam, stellen bekanntermaßen Einsatzstoffe für die Herstellung technisch bedeutender Kunststoffe wie Nylon dar. Cyclische Amine, wie Azepan, finden vielfältige Verwendung als Zwischenprodukte für die Herstellung von Pharma- und Agrochemikalien, Korrosionsinhibitoren für Nichteisenmetalle, Vulkanisationsbeschleuniger und als Bestandteil von Textilhilfsmitteln und -schlichten, Antistatika und Ausrüstungsmittel sowie Vernetzungsmitteln von Harzen.

Aus GB 1 358 862 (1974) ist die Herstellung von Lactamen aus insbesondere fünf- und höhergliedrigen Azacycloalkanen oder aus Diaminen und Wasser an festen Hydrierkatalysatoren bei 150-400°C in flüssiger Phase bekannt.

Dabei wird an Ra-Ni, Pt/C und Ru/Al₂O₃ aus Piperidin/H₂O/NH₃ im Gewichtsverhältnis 1/10/9 (Molverhältnis ca. 1/47/45) bei 300°C (2-3 Stunden) Piperidon in Ausbeuten um 50% erhalten. Dagegen werden aus Azepan ("Hexamethylenimin", HMI)/H₂O/NH₃ ebenfalls im Gewichtsverhältnis 1/10/9 bei 270°C an Ra-Ni (5 Stunden) nur 17.6% der theoretischen Menge an Caprolactam gebildet.

Setzt man als Diamin Hexamethylendiamin ("HMD") ein, so liegt die Ausbeute an Caprolactam mit 10.7 % noch niedriger als die Ausbeute aus Azepan und damit weit unter dem technisch erforderlichen Niveau, wobei Azepan bei dieser Umsetzung offenbar nicht entsteht.

Verfahren zur Herstellung von Azacycloalkanen aus Diaminen, wie Azepan aus HMD, ohne die gleichzeitige Herstellung von cyclischen Lactamen sind allgemein bekannt.

So beschreibt CA-A 920 606 die Umwandlung von HMD in Gegenwart von H₂ bei HMD/H₂ = 1:2-70, 150-250°C und 1-20 bar an Co- und Ni-Katalysatoren (Ra-Typ sowie auf Trägern wie SiO₂, Al₂O₃) zu Azepan.

Bei unvollständigen Umsätzen (bis 44%) erreicht man Azepan-Selektivitäten bis ca. 90%. Als Nebenprodukte entstehen vorwiegend Bis-hexamethylen-triamin und Polyamine.

Nach US-A 3,830,800 erzielt man aus HMD in einem Lösungsmittel wie Dioxan an RuO₂/C ebenfalls nur bei niedrigen Umsätzen (<50%) gute Azepan-Selektivität (91%).

DE-A 24 14 930 beschreibt die HMD-Kondensation an Metallen aus der Gruppe bestehend aus Ni, Co, Fe, Mn, Ag, Cu, Pd als wirksamen Komponenten, auch auf Trägern wie Al₂O₃ oder SiO₂, in einem hochsiedenden Lösungsmittel bei Temperaturen von 200°C unter gleichzeitig kontinuierlichem Abdestillieren des entstehenden Azepans (Sdp. 139°C bei Normaldruck) aus dem Reaktionsgemisch. Ausbeuten bis zu 94% wurden gefunden.

DE-A 25 32 871 betrifft die kontinuierliche Kondensation von HMD in einem inerten Lösungsmittel an Ni oder Co, auch auf Trägern wie Al₂O₃ oder SiO₂, bei Temperaturen von 80-150°C, wobei zur Verhinderung der Bildung von Oligo- und Polyaminen das Azepan kontinuierlich aus dem Reaktionsgemisch durch Azeotropdestillation mit H₂O entfernt wird.

Aus US-A 3,903,079 ist bekannt, daß bei der Kondensation von HMD bei Einsatz von HMD/NH₃ = 1:15-30 (1:>2) bei 250-400°C an Zeolithen, beladen mit 0.3-7% Metallkationen aus der Gruppe bestehend aus Cu, Pd, Mn, Ni oder Cr in der Gasphase im Festbett- oder Wirbelreaktor Azepan mit Ausbeuten um 75% erhalten werden kann.

US-B 470,900 beschreibt die Gasphasenkondensation von Diaminen zu Azacycloalkanen, darunter von HMD zu Azepan, bei 100-250°C an Ni, Co, Fe oder Kupferkatalysatoren auf Trägern ohne Einsatz von NH₃.

Bei einem HMD/H₂ -Verhältnis von 1:20, 150°C und einer Raumgeschwindigkeit von 0.2 wurden an Ni/Kieselgur Azepan-Ausbeuten von 90% erreicht, mit Cu/Kieselgur wurde bei einem bei einem HMD/H₂ -Verhältnis von 1:20, 150°C und einer Raumgeschwindigkeit von 0.1 eine Azepan-Ausbeute von 95% erreicht.

Aus EP-A 372 492 ist die Azepan-Herstellung aus HMD bei 160-260°C in Gegenwart von Wasserdampf und Wasserstoff an Pd/Al₂O₃ in der Gasphase bei einem Gewichtsverhältnis von HMD zu Wasser von 20:80 bis 99:1 bekannt. Hierbei wurden Azepan-Ausbeuten von 92 % erzielt.

Verfahren zur Umsetzung von cyclischen Aminen, wie Azepan, zu cyclischen Lactamen, wie Caprolactam, sind ebenfalls bekannt.

Gemäß Chem. Ber. 109 (1976) 3707-27 kann Pyrrolidin mit Sauerstoff unter Pt-Katalyse in Ausbeuten von 60 % zu Pyrrolidon umgesetzt werden, für andere cyclische Amine führt die entsprechende Umsetzung hingegen zu einem vollkommen unübersichtlichen Produktgemisch.

US-A 3,634,346 beschreibt die Umsetzung eines cyclischen Amins zu dem entsprechenden cyclischen Lactam durch Oxidation mit einem Hydroperoxid in Gegenwart eines Metall-Ionen-Katalysators. Die besten Ausbeuten, die mit diesem Verfahren erhalten werden können (15.5 % 2-Pyrrolidon aus Pyrrolidin), sind aber für industrielle Verfahren gänzlich unbefriedigend.

Verfahren zur Oxidation von cyclischen Aminen zu den entsprechenden cyclischen Lactamen mit Hg-(II)-Verbindungen, wie sie aus US-A-3,336,299 und Synth. Commun. 18 (1988) 1331-37 bekannt sind, sind hinsichtlich der Ausbeute vollkommen unbefriedigend und bezüglich der Aufarbeitung und Entsorgung der Hg-haltigen Reaktionsrückstande problematisch.

Dies gilt gleichermaßen für die Oxidation mit Iodosobenzol, wie in Tetrahedron Lett. 29 (1988) 6913-16 beschrieben.

Verfahren zur Herstellung cyclischer Lactame, wie Caprolactam, aus aliphatischen alpha, omega-Aminonitrilen, wie 6-Aminocapronitril, sind ebenfalls bekannt.

US-A 2,357,484 beschreibt die Umsetzung von 6-Aminocapronitril bei 200 bis 350°C zu Caprolactam in Gegenwart heterogener Katalysatoren. Hierbei werden Raum-Zeit-Ausbeuten von 0.02 bis 0.03 g (Produkt) / ml (Katalysator) / h erzielt.

Aus EP-A 150 295 und US-A 4,628,085 ist die Umsetzung von Aminonitrilen zu cyclischen Lactamen bei 200 bis 400°C in Gegenwart von Katalysatoren bekannt. Das Eduktgemisch enthielt dabei nur bis zu 4 % Aminonitril.

WO-A 96/22974 beschreibt die Umsetzung von 6-Aminocapronitril zu Caprolactam bei 200 bis 450°C an Katalysatoren, die in US-A 4,628,085 im Falle des Siliziumdioxids als sehr kurzlebig beschrieben sind.

Aus DE-A 19 632 006 ist bekannt, 6-Amincapronitril zu Caprolactam bei 220 bis 380 in Gegenwart von heterogenen Katalysatoren umzusetzen. Die Katalysatorbelastungen betrugen dabei 0,1 bis 1 g (Edukt) / ml (Katalysator) / h.

DE 915 568 C und DE 765 203 C beschreiben Verfahren zur gemeinsamen Herstellung sieben- bzw. sechsgliedriger cyclischer Lactame und cyclischer Amine ausgehend von γ- bzw. δ-Cyancarbonsäure (oder ihrer Ester oder Amide).

DE 195 00 222 A beschreibt ein Verfahren zur Herstellung von Caprolactam und Hexamethylendiamin ausgehend von Adipodinitril, wobei 6-Aminocapronitril als Zwischenprodukt entsteht (in einer Mischung mit Hexamethylendiamin, Hexamethylenimin und anderen Verbindungen).

Aufgabe der vorliegenden Erfindung war es, ein Verfahren zur Verfügung zu stellen, das die gleichzeitige Umsetzung eines aliphatischen alpha, omega-Diamins und eines aliphatischen alpha, omega-Aminonitrils zu einem cyclischen Amin und einem cyclischen Lactam auf technisch einfache und wirtschaftliche Weise ermöglicht.

Demgemäß wurde das eingangs definierte Verfahren gefunden.

Als Ausgangsstoffe im erfindungsgemäßen Verfahren können vorzugsweise aliphatische alpha, omega-Diamine der allgemeinen Formel (I)

H₂N―(CH₂)ₙ―NH₂ (I)

in der n einen Wert von 4, 5, 6 oder 7 hat, d.h. 1,4-Diaminobutan, 1,5-Diaminopentan, 1,6-Diaminohexan ("Hexamethylendiamin", "HMD") und 1,7-Diaminoheptan, ganz besonders bevorzugt 1,6-Diaminohexan, oder Gemische solcher Diamine (I).

Die Diamine können dabei am Kohlenstoff eine oder mehrere Substituenten, wie C₁ - bis C₆ - Alkyl-Gruppen, Cycloalkyl-Gruppen, wie Cyclopentyl-, Cyclohexyl, oder Cycloheptyl-Gruppen, oder Halogen, tragen. Vorzugsweise sind die Diamine nicht substituert.

Solche Diamine und Verfahren zu deren Herstellung sind allgemein bekannt.

Als aliphatische alpha, omega-Aminonitrile kommen im erfindungsgemäßen Verfahren in erster Linie solche der allgemeinen Formel (II)

H₂N―(CH₂)ₘ―CN (II)

in Betracht, in der m einen Wert von 3, 4, 5, oder 6 hat, d.h. 4-Aminobutyronitril, 5-Aminovaleronitril, 6-Aminocapronitril ("ACN") und 7-Aminooenanthonitril, ganz besonders bevorzugt 6-Aminocapronitril, oder Gemische solcher Aminonitrile (II).

Die Aminonitrile können dabei am Kohlenstoff eine oder mehrere Substituenten, wie C₁ - bis C₆ - Alkyl-Gruppen, Cycloalkyl-Gruppen; wie Cyclopentyl-, Cyclohexyl, oder Cycloheptyl-Gruppen, oder Halogen, tragen. Vorzugsweise sind die Aminonitrile nicht substituert.

Solche Aminonitrile und Verfahren zu deren Herstellung sind allgemein bekannt und kommerziell verfügbar.

Bevorzugte Mischungen aus einem Diamin (I) und einem Aminonitril (II) sind solche, für die n = m+1 gilt. Vorteilhaft kommen Mischungen in Betracht mit einem molaren Verhältnis von Aminonitril (II) zu Diamin (I) von 5 : 95 bis 90 : 10, vorzugsweise von 10 : 90 bis 80 : 20, insbesondere 30 : 70 bis 60 : 40.

Erfindungsgemäß führt man die Umsetzung in Gegenwart eines heterogenen Katalysators oder Gemische solcher Katalysatoren durch.

Als heterogene Katalysatoren kommen vorzugsweise solche in Betracht, die
(a) eine Dehydrierkomponente (III) und
(b) eine acide und/oder amphotere Komponente (IV) enthalten.

Als Dehydrierkomponente (III) kommt ein Metall aus der Gruppe bestehend aus Cu, Ag, Ni, Co, Pd, Pt, Rh, Ru, Ir, Os und Re, vorzugsweise aus der Gruppe bestehend aus Cu, Ag, Ni, Co, Pd, Pt, Rh und Ru, insbesondere aus der Gruppe bestehend aus Cu, Co und Ru, oder deren Gemische in Betracht.

Als Komponente (IV) können vorteilhaft Oxide, Oxidhydrate, Silikate, Phosphate, Heteropolysäuren oder acide Zeolithe der Metalle Mg, Al, B, Ti, Zr, Hf, V, Nb, Ta, Mo, W, Fe, Cr, Ge, Zn, Sn, Bi, Th, U oder der Lanthaniden wie La und Ce, vorzugsweise SiO₂, gamma-Al₂O₃, Nb-, Ta-, Zr-Oxide sowie Phsophate von La, Nb, Zr, Al und B, oder deren Gemische eingesetzt werden, wobei die Acidität dieser Verbindungen durch Dotierung mit organischen oder vorzugsweise anorganischen Säuren wie Phosphorsäure, Schwefelsäure oder Halogenwasserstoffsäuren gegebenenfalls erhöht werden kann.

Die Dehydrierkomponente (III) kann als Vollkatalysator, beispielsweise als Raney-Ni oder Raney-Co oder vorzugsweise als Trägerkatalysator eingesetzt werden. Als Trägermaterial kommt dabei ein inertes Material, wie C, Steatit oder alpha-Al₂O₃, in Betracht, vorzugsweise eine acide und/oder amphotere Komponente, wie eine Komponente (IV), insbesondere SiO₂, gamma-Al₂O₃, Nb-, Ta-, Zr-Oxide sowie Phsophate von La, Nb, Zr, Al und B, oder deren Gemische.

Die Mengenverhältnisse von Dehydrierkomponente (III) zu Komponente (IV) kann von einem Fachmann nach den gewünschten Reaktionsparametern, wie Produktverhältnis oder Katalysatoraktivität, durch einige einfache Vorversuche leicht und ohne großen Aufwand ermittelt werden. Im allgemeinen kommen Gewichtsverhältnisse von Komponente (III) zu Komponente (IV) von 0.1 : 99.9 bis 50 : 50, vorzugsweise 0.5 : 99.5 bis 15 : 85 in Betracht.

Erfindungsgemäß setzt man in dem Verfahren Wasser ein, vorteilhaft in einem molaren Verhältnis zu der Summe aus Diamin (I) und Aminonitril (II) von größer als 1, vorzugsweise in einem molaren Verhältnis von Wasser zu der Summe aus Diamin (I) und Aminonitril (II) von 3 : 1 bis 25 : 1, insbesondere 5 : 1 bis 20 : 1.

Vorteilhaft kann man dem Reaktionsgemisch ein Intergas, wie Stickstoff, Argon, CO oder Methan, zugeben, vorzugsweise in einem molaren Verhältnis von Inertgas zu der Summe aus Diamin (I) und Aminonitril (II) von 5 : 1 bis 100 : 1, wobei das molare Verhältnis von Inertgas zu der Summe aus Diamin (I) und Aminonitril (II) vorteilhaft umso geringer sein kann, je größer das molare Verhältnis von Wasser zu der Summe aus Diamin (I) und Aminonitril (II) ist.

Ebenfalls vorteilhaft kann man als Inertgas Wasserstoff dem Reaktionsgemisch zugeben, der nach den bisherigen Erfahrungen einen standzeitverlängernden Effekt auf den heterogenen Katalysator aufweist, vorzugsweise in einem molaren Verhältnis von Wasserstoff zu Diamin (I) von 0.01 : 1 bis 10 : 1, insbesondere 0.01 : 1 bis 5 : 1. Dabei kann durch eine Erhöhung des molaren Verhältnisses von Wasserstoff zu Diamin (I) insbesondere im Falle von sehr hydrieraktiven Komponenten (III), wie Ni, Pt oder Pd, das Produktverhältnis von cyclischem Amin zu cyclischem Lactam zugunsten des cyclischen Amins verschoben werden.

Das erfindungsgemäße Verfahren kann man vorteilhaft bei 200 bis 600°C, vorzugsweise 240 bis 350°C bei Drücken von 0,1 bis 2 bar, vorzugsweise 0,75 bis 1,5 bar, insbesondere 0,9 bis 1,1 bar in einem üblichen hierfür geeigneten Reaktor, wie einem Festbettreaktor oder einem Wirbelbettreaktor, durchführen.

Die Aufarbeitung der Produktmischung kann in an sich bekannter Weise, beispielweise extraktiv und/oder vorzugsweise destillativ, erfolgen.

Erfindungsgemäß erhält man bei dem Verfahren gleichzeitig ein cyclisches Lactam, vorzugsweise ein cyclisches Lactam der Formel (Va) bzw. (Vb) 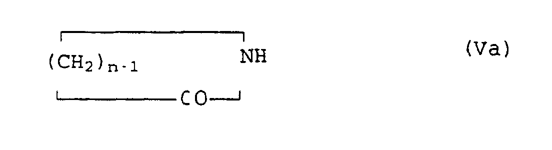 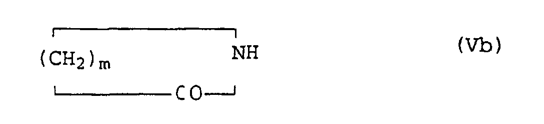 oder ein Gemisch solcher Lactame zusammen mit einem cyclischen Amin vorzugsweise einem cyclischen Amin der Formeln (VIa) bzw. (VIb) 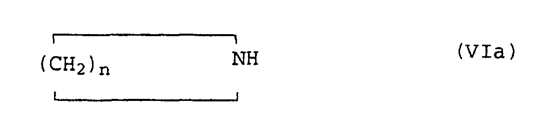 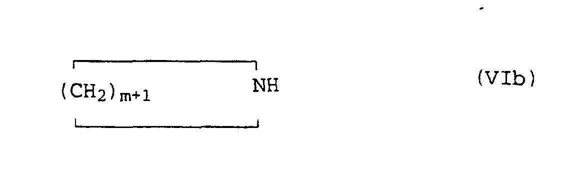 oder einem Gemisch solcher Amine,
wobei in den Formeln (Va) und (VIa) n eine ganze Zahl von 4 bis 7 und in den Formeln (Vb) und (VIb) m eine ganze Zahl von 3 bis 6 bedeutet.

Besonders bevorzugte Lactame (Va) sind solche, in denen n einen Wert von 4, 5, 6, und 7 hat, insbesondere 6, und Lactame (Vb) solche, in denen m einen Wert von 3, 4, 5 und 6 hat, d.h. Azolan-2-on ("2-Pyrrolidon", "gamma-Butyrolactam"), Azixan-2-on ("Piperidin-2-on", "Valerolactam"), Azepan-2-on ("Caprolactam") und Azacyclooctan-2-on, ganz besonders bevorzugt Caprolactam, oder Gemische solcher Lactame (Va) bzw. (Vb).

Besonders bevorzugte Amine (VIa) sind solche, in denen n einen Wert von 4, 5, 6 und 7 hat, insbesondere 6, und Amine (VIb) solche, in denen m einen Wert von 3, 4, 5 und 6 hat, d.h. Azolan ("Pyrrolidin"), Azixan ("Piperidin"), Azepan ("Hexamethylenimin", "HMI"), Azacyclooctan, ganz besonders bevorzugt Azepan, oder Gemische solcher Amine (VIa) bzw. (VIb).

Setzt man vorzugsweise Ausgangsverbindungen mit n = m+1 ein, so sind vorteilhaft die Lactame (Va) und (Vb) identisch wie auch die Amine (VIa) und (VIb).

Das bei dem erfindungsgemäßen Verfahren erhaltene Amin (VI) kann neben den bereits genannten technischen Verwendungen auch zu dem entsprechenden Lactam (V) umgesetzt werden, vorzugsweise durch Rückführung in das erfindungsgemäße Verfahren, insbesondere im Gemisch mit dem entsprechenden aliphatischen alpha, omega-Diamin (I) und aliphatischen alpha, omega-Aminonitril.

Die Umsetzung kann dabei vorteilhaft unter den für das erfindungsgemäße Verfahren genannten Reaktions- und Verfahrensbedingungen in Gegenwart des genannten heterogenen Katalysators erfolgen.

### Beispiel

Eine wäßrige Lösung enthaltend 50 Gew.-% einer Mischung aus HMD und ACN im molaren Verhältnis von 1.9 : 1 wurde verdampft und unter Zufuhr von 10 l H₂/h (100 l/l (Katalysator)·h) mit einer Belastung von 100 g/l (Katalysator)·h (entsprechend 50g der Summe aus HMD und ACN pro Liter und Stunde) in Abwärtsfahrweise bei 275°C über 100 ml Cu/Al₂O₃ (3 mm-Stränge, Länge der Stränge 0,5 bis 1,5 cm, Reaktor-Innendurchmesser 29 mm) geleitet.

Der in Kühlfallen kondensierte Austrag enthielt nach GC-Analyse bei Umsätzen von 91,2 % HMD und 93,9 % ACN Caprolactam mit einer Selektivität von 64,7 % bezogen auf die Summe der Einsatzstoffe und Azepan mit einer Selektivität von 28,1 % bezogen auf die Summe der Einsatzstoffe.

## Patentansprüche

1. Verfahren zur gemeinsamen Herstellung eines cyclischen Lactams und eines cyclischen Amins durch gleichzeitige Umsetzung eines aliphatischen alpha, omega-Diamins und eines aliphatischen alpha, omega-Aminonitrils in der Gasphase mit Wasser in der Gegenwart eines heterogenen Katalysators, wobei das Molverhältnis von Wasser zu der Summe aus aliphatischem alpha, omega-Diamin und aliphatischem alpha, omega-Aminonitril 3 bis 25 ist, das molare Verhältnis von dem aliphatischen alpha, omega-Aminonitril zu dem aliphatischen alpha, omega-Diamin zwischen 5 : 95 und 90 : 10 liegt und der heterogene Katalysator
(a) eine Dehydrierkomponente und
(b) eine acide und/oder amphotere Komponente enthält.

2. Verfahren nach Anspruch 1, wobei man als aliphatisches alpha, omega-Diamin Hexamethylendiamin und als aliphatisches alpha, omega-Aminonitril 6-Aminocapronitril einsetzt unter Erhalt von Azepan als cyclisches Amin und Caprolactam als cyclisches Lactam.

3. Verfahren nach Anspruch 1 oder 2, wobei die Temperatur 200 bis 600°C beträgt.

4. Verfahren nach den Ansprüchen 1 bis 3, wobei der heterogene Katalysator als Dehydrierkomponente ein Metall aus der Gruppe bestehend aus Cu, Ag, Ni, Co, Pd, Pt, Rh, Ru, Ir, Os und Re enthält.

5. Verfahren nach Anspruch 4, wobei der heterogene Katalysator als Dehydrierkomponente ein Metall aus der Gruppe bestehend aus Cu, Co und Ru enthält.

## Claims

1. The process for coproducing a cyclic lactam and a cyclic amine by coreacting an aliphatic alpha, omega-diamine and an aliphatic alpha, omega-aminonitrile with water in the gas phase in the presence of a heterogeneous catalyst by using a molar ratio of water to the sum total of aliphatic alpha, omega-diamine and aliphatic alpha, omega-aminonitrile within the range from 3 to 25, a molar ratio of aliphatic alpha, omega-aminonitrile to aliphatic alpha, omega-diamine within the range from 5 : 95 to 90 : 10 and a heterogeneous catalyst comprising
(a) a dehydrogenating component and
(b) an acidic and/or amphoteric component.

2. The process of claim 1, wherein hexamethylenediamine and 6-aminocapronitrile are used as the aliphatic alpha, omega-diamine and the aliphatic alpha, omega-aminonitrile, respectively, to obtain azepan as the cyclic amine and caprolactam as the cyclic lactam.

3. The process of claim 1 or 2, wherein the temperature is within the range from 200 to 600°C.

4. The process of any of claims 1 to 3, wherein the dehydrogenating component in the heterogeneous catalyst is a metal selected from the group consisting of Cu, Ag, Ni, Co, Pd, Pt, Rh, Ru, Ir, Os and Re.

5. The process of claim 4, wherein the dehydrogenating component in the heterogeneous catalyst is a metal selected from the group consisting of Cu, Co and Ru.

## Revendications

1. Procédé de préparation conjointe d'un lactame cyclique et d'une amine cyclique par réaction simultanée d'une alpha,oméga-diamine aliphatique et d'un alpha,oméga-aminonitrile aliphatique dans la phase gazeuse avec de l'eau, en présence d'un catalyseur hétérogène, procédé dans lequel le rapport molaire entre l'eau et la somme de l'alpha,oméga-diamine aliphatique et de l'alpha,oméga-aminonitrile aliphatique est de 3 à 25, le rapport molaire entre l'alpha,oméga-amino-nitrile aliphatique et l'alpha,oméga-diamine aliphatique est compris entre 5/95 et 90/10, et le catalyseur hétérogène contient
(a) un composant de déshydrogénation et
(b) un composant acide et/ou amphotère.

2. Procédé suivant la revendication 1, dans lequel on met en oeuvre, comme alpha,oméga-diamine aliphatique, de l'hexaméthylènediamine et, comme alpha,oméga-aminonitrile aliphatique, du 6-aminocapronitrile, avec obtention d'azépan, comme amine cyclique, et de caprolactame, comme lactame cyclique.

3. Procédé suivant l'une des revendications 1 et 2, dans lequel la température est de 200 à 600°C.

4. Procédé suivant l'une des revendications 1 à 3, dans lequel le catalyseur hétérogène contient, comme composant de déshydrogénation, un métal du groupe constitué de Cu, Ag, Ni, Co, Pd, Pt, Rh, Ru, Ir, Os et Re.

5. Procédé suivant la revendication 4, dans lequel le catalyseur hétérogène contient, comme composant de déshydrogénation, un métal du groupe constitué de Cu, Co et Ru.
